(19) Europäisches Patentamt
European Patent Office
Office européen des brevets

(11) **EP 3 538 896 B1**

(12) **EUROPEAN PATENT SPECIFICATION**

(45) Date of publication and mention
of the grant of the patent:
**19.07.2023 Bulletin 2023/29**

(21) Application number: **16921351.9**

(22) Date of filing: **14.11.2016**

(51) International Patent Classification (IPC):
**G01N 33/74** (2006.01)   **G01N 35/00** (2006.01)
**G01N 33/68** (2006.01)

(52) Cooperative Patent Classification (CPC):
**G01N 33/56922; G01N 33/6893; G16Z 99/00;**
G01N 2333/205; G01N 2800/062; Y02A 90/10

(86) International application number:
**PCT/FI2016/050800**

(87) International publication number:
**WO 2018/087419 (17.05.2018 Gazette 2018/20)**

(54) **IMPROVED METHOD FOR DETECTION OF HELICOBACTER PYLORI -GASTRITIS AND ATROPHIC GASTRITIS WITH RELATED RISKS**

VERBESSERTES VERFAHREN ZUM NACHWEIS VON HELICOBACTER-PYLORI-GASTRITIS UND ATROPHISCHER GASTRITIS MIT VERBUNDENEN RISIKEN

PROCÉDÉ AMÉLIORÉ POUR LA DÉTECTION D'UNE GASTRITE À HELICOBACTER PYLORI ET D'UNE GASTRITE ATROPHIQUE AVEC DES RISQUES ASSOCIÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Validation States:
**MA**

(43) Date of publication of application:
**18.09.2019 Bulletin 2019/38**

(73) Proprietor: **BIOHIT OYJ**
**00880 Helsinki (FI)**

(72) Inventor: **SUOVANIEMI, Osmo**
**00570 Helsinki (FI)**

(74) Representative: **Laine IP Oy**
**Porkkalankatu 24**
**00180 Helsinki (FI)**

(56) References cited:
**EP-B1- 1 454 134          EP-B1- 1 454 134**
**WO-A1-2005/108426     WO-A1-2005/108426**
**WO-A1-2008/025877     WO-A1-2008/025877**
**WO-A1-2009/053537     WO-A1-2015/189480**

- **Pia Rinkinen: "GASTRIN-17", , 3 December 2013 (2013-12-03), XP055475254, Retrieved from the Internet: URL:http://www.biohithealthcare.com/resource/files/media/manuals/g17-ifu.pdf [retrieved on 2018-05-15]**
- **CHRISTOPHER M M ET AL: "Effect of Specimen Collection and Storage on Blood Glucose and Lactate Concentrations in Healthy, Hyperthyroid and Diabetic Cats", VETERINARY CLINICAL PATHOLOGY, VETERINARY PRACTICE PUBLISHING, MISSION VIEJO, CA, US, vol. 29, no. 1, 1 January 2000 (2000-01-01), pages 22-28, XP002264424, ISSN: 0275-6282**
- **DONNELLY J G ET AL: "STABILITY OF TWENTY-FIVE ANALYTES IN HUMAN SERUM AT 22°C, 4°C, AND-20°C", PEDIATRIC PATHOLOGY AND LABORATORY MEDICINE, TAYLOR & FRANCIS, WASHINGTON, DC, US, vol. 15, no. 6, 1 January 1995 (1995-01-01), pages 869-874, XP008025299, ISSN: 1077-1042**
- **EASTVOLD, M. L. ET AL.: 'A two-center international evaluation of the Immulite 2000 automated serum gastrin assay' CLINICAL BIOCHEMISTRY vol. 39, no. 4, April 2006, pages 387 - 390, XP027878022**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD

The present invention relates to methods for examining a person having the biomarkers indicating *Helicobacter pylori* infection and/or atrophic gastritis with related risks. More precisely, the present invention relates to methods for improving the analysis steps relating to the screening and detection of the previously mentioned diseases and function of stomach mucosa.

BACKGROUND

GastroPanel®, the unique *Helicobacter pylori* test can detect the following conditions:

1) *Helicobacter pylori* (HP) infection which is an independent risk factor of both gastric cancer and peptic ulcer disease (gastric- and duodenal ulcer).

2) HP infection -induced atrophic gastritis (AG), which in most cases is asymptomatic, as well as the topographic site of AG either in the corpus or in antrum. Apart from HP infection, AG in the corpus with all its clinical sequels can also develop through an autoimmune disease of the gastric mucosa.

2.1) AG of the corpus mucosa leads to low acid output or eventually acid-free (achlorhydric) stomach. This increases the risk of gastric or esophageal cancer, as well as malabsorption of vitamin B12, calcium, magnesium and zinc. In addition, absorption of some medicines, e.g. dipyridamol, some iron preparations and anti-fungal drugs (fluconazol, itraconazol), thyroxin and atazanovir is impaired due to acid free stomach. Calcium deficiency can cause osteoporosis, and vitamin B12 deficiency can contribute to development of Alzheimer's disease, dementia, depression or peripheral neuropathies. Reduced acid output in the stomach can also increase the risk of serious infections in the gastrointestinal- and respiratory tract, including giardiasis, malaria, Clostridium difficile, *E. coli* EHEC and pneumonia.

2.2) AG of the antrum that increases the risk of gastric cancer. Co-existent AG of the corpus and antrum (pangastritis) is the single most important risk condition for gastric cancer.

3) HP infection also in subjects with AG, MALT-lymphoma or bleeding peptic ulcer, and in those taking PPI medication or antibiotics. In these cases, $^{13}$C Urea Breath Test (UBT) or stool HP antigen test frequently give false negative results, and HP infection (with all its possible consequences) remains undetected. UBT may give false positive results in subjects with acid-free stomach. In addition, UBT and HP antigen or antibody tests do not detect AG due to HP-infection or autoimmune disease (http://www.biohithealthcare.com/limitations-of-helicobacter-pylori-diagnostics ).

4) High acid output of the gastric mucosa, which predisposes to esophageal reflux disease with potential complications. These are ulcerative esophagitis, Barrett's esophagus or lower esophageal cancer.

Symptomatic *Helicobacter pylori* (HP) infection, atrophic gastritis (AG) and high acid output are indications for gastroscopy.

The GastroPanel® innovation is based on follow-up studies on gastritis patients conducted by research groups in Finland and Estonia and the discovery of the role of *Helicobacter pylori* in pathogenesis of gastritis and peptic ulcer disease, which led to Nobel Prize in 2005, as well as on Biohit's R&D and the microplate immunoassay analyzers based on the invention of the vertical light beam measurement principle.

More precisely, BIOHIT GastroPanel® is a quantitative ELISA test panel that measures the blood plasma concentration of four biological markers of gastric mucosal structure and function: pepsinogen I (PGI), pepsinogen II (PGII), gastrin-17 (G-17) and *Helicobacter pylori* IgG antibodies. The main indications of use for GastroPanel® are to aid in the diagnosis of and for screening of asymptomatic and symptomatic subjects with 1) *H. pylori* infection 2) atrophic gastritis (AG) 3) disturbed gastric function.

GastroPanel® is the first-line diagnostic test for *Helicobacter pylori (Hp)* infection (5-80% of the world population), for examination of all the patients with dyspepsia (20-40% of the western population), as well as for the screening of atrophic gastritis (AG) with related risks, such as stomach- and oesophageal cancer (1-3). Atrophic gastritis also enhances the risk of malabsorption of vitamin B12, iron, magnesium, zinc, calcium and some medicines. The $^{13}$C Urea Breath Test (UBT), stool antigen test and antibody tests for *Hp* infection do not detect atrophic gastritis which is caused by *Hp* infection or an autoimmune disease nor the level of acid output. In addition, the $^{13}$C Urea Breath Test and stool antigen test may give false negative results if the patient has a) atrophic gastritis b) MALT lymphoma c) bleeding peptic ulcer disease or d) if the patient is currently receiving antibiotics or PPIs.

GastroPanel® consists of key stomach-specific biomarkers representing the key regulators of normal stomach physiology. These four biomarkers include pepsinogen I (PGI), pepsinogen II (PGII), amidated gastrin-17 (G-17), and *Hp*-antibodies, designed to give information on both the structure and function of the stomach mucosa (1-6). Most importantly, this panel gives accurate estimates of the capacity of the corpus and antrum mucosa to secrete gastric acid and G-17, respectively, as well as of important gastric pathologies, like inflammation, grade and topography of atrophic gastritis (7-9), which may represent increased risk of gastric cancer and its clinical sequels (1).

Normal plasma levels of all four biomarkers indicate that the stomach mucosa has normal structure and function, whereas the abnormal levels are signs of a non-healthy stomach, reflecting the disturbances in the feedback mechanisms between the acid output of the corpus, PGs and G-17. For G-17 assessment, there are two options; G-17 basal (G-17b) values, and G-17 stimulated (G-17s) values, the latter being particularly important in distinguishing between functional disturbance of the antrum (G-17s normal) and AG in the antrum (G-17s does not increase in AG)(10,1 1).

Being the first non-invasive diagnostic test for stomach mucosal health, GastroPanel® is unique also in that the results are interpreted by a software application (GastroSoft) (http://www.gastropanel.com), specifically designed for this purpose. GastroPanel® results are classified into one of five possible diagnostic categories: 1) normal mucosa, 2) superficial (*Hp*) gastritis, 3) AG in the antrum, 4) AG in the corpus, and 5) AG in both antrum and corpus (pangastritis)(11,12). Thus, GastroPanel® is optimized for use together with the Updated Sydney System (USS) for classification of gastritis, which is based on these same 5 diagnostic categories (13).

GastroPanel® has been validated in several large trials based on biopsy-confirmed gastroscopies (14,15), all included in a recent meta-analysis on the subject (14). These studies have been exploited to establish the validated reference (cut-off) values for each individual biomarkers of the panel, using the five histological categories as endpoints. These studies confirm the high accuracy of GastroPanel in detecting the most important study endpoint, moderate-to-severe AG (14-16). Thus, normal values of PGI, PGII and their ratio (PGI/PGII) preclude AG of the corpus with NPV over 95%. In turn, the values of PGI and PGII as well as their ratio below the established cut-off levels predict moderate-to-severe AG with area under ROC curve (AUC) values above 0.950 in adequately powered and USS validated series (1,2,3,16,17).

In brief, the levels of PGI decrease in AG of the corpus (and in pan-gastritis), but remain within the normal range in all other conditions. Elevated PGII levels reflect mucosal inflammation, the highest values being detected in *Hp*-associated non-AG. The G-17b values are highest in AG of the corpus, because of the missing negative feedback by the acid output from an atrophic corpus, resulting in uninhibited secretion of G-17b by the normal antral mucosa. The same applies to the situation where acid output is inhibited by prolonged use of PPI medication. By definition, when antral mucosa is atrophic and the G cells are depleted, G-17 secretion remains very low even after protein stimulation (G-17s)(17).

*Hp* IgG antibodies give significant added diagnostic value to the three biomarkers. IgG serology for *Hp* measures potentially two different conditions: 1) an ongoing *Hp*-infection, or 2) a previous exposure to *Hp*. Although GastroPanel® is not capable of making the distinction between these two, the evidence on *Hp* involvement gives significant added diagnostic value. As the only abnormal marker, *Hp* implicates an *Hp*-associated superficial gastritis (with no AG), while associated with abnormalities in the other three markers, elevated *Hp*-antibody titers confirm the diagnosis of *Hp*-associated AG (antrum or corpus)(1,3,18,19).

For more details in interpretation of the GastroPanel® results, please refer to http://www.gastropanel.com.

Also, WO 2009/053537 describes the principle methods and concepts relating to the GastroPanel innovation.

Furthermore, Syrjänen (2016) provides systematic review and meta-analysis of all relevant studies on GastroPanel® in diagnosis of atrophic gastritis and corroborates the statement of international experts, advocating GastoPanel® in diagnosis and screening of AG. Due to its high specificity for both AG of the antrum and AG of the corpus, GastroPanel® is truly a test for stomach health.

In the GastroPanel® test concentrations of four biomarkers are determined from a blood sample. These biomarkers: pepsinogen I (PGI), pepsinogen II (PGII) and gastrin-17 (G17), which are secreted by the cells in gastric mucosa, are measured. Additionally, *Helicobacter pylori* antibodies are measured. An overall interpretation of all four biomarker results provides more reliable and comprehensive understanding of the structure and function of the gastric mucosa than what could be achieved by using the individual biomarkers separately. The concentrations of these biomarkers should however be determined as soon as possible, or to take care that the samples are properly stored and handled before analyses. It is recommended that the blood sample should be centrifuged within 30 minutes from collection of the sample. A special stabilizer substance should be added to the separated plasma tube to prevent degradation of any of the analytes. The stabilizer enables the storage of the sample at room temperature (20-25 °C) for three days, or in the refrigerator (2-8 °C) for up to seven days. In case of longer delay before analysis, the sample tolerates storage at -20 °C for up to 2 weeks. For any longer storage time, the sample should be kept at -70 °C. However, if it is not possible to centrifuge the sample it is not possible to use the stabilizer as the red blood cells become haemolysed. Therefore, the effect of temperature and time during storage and transportation of the whole blood sample is of high interest.

SUMMARY OF THE INVENTION

The invention is defined by the features of the independent claims. Some specific embodiments are defined in the dependent claims.

According to a first aspect of the present invention, there is provided a method for screening of asymptomatic and symptomatic subjects with *Helicobacter pylori* infection, atrophic gastritis with related risks and/or disturbed gastric function, based on a whole blood sample obtained from a subject, recording of sample collection time and sample storage temperature, measuring biomarker concentration of interest, and determining an initial biomarker concentration and status of stomach mucosa based on such information.

According to a second aspect of the present invention, the initial concentration of a gastrin-17 biomarker, determined by such means, is used in a non-invasive test for screening of asymptomatic and symptomatic subjects with *Helicobacter pylori* infection, atrophic gastritis with related risks and/or disturbed gastric function.

The present invention is based on the finding that the concentration of gastrin-17 biomarker drops monotonically versus time and temperature. If the concentration is higher than the limit of quantification and the information of the storage temperature and time of sample collection is known, the initial gastrin-17 concentration can be reliably estimated and used for interpreting the results of stomach mucosa health.

These and other aspects, together with the advantages thereof over known solutions are achieved by the present invention, as hereinafter described and claimed.

More precisely, the method of the present invention is characterized by what is stated in claim 1.

Considerable advantages are obtained by means of the invention. For example, blood samples do not require immediate centrifugation for separation of plasma or serum for further analysis. Instead, samples can be collected, stored and sent for further analysis as whole blood samples without processing them first. In the analysis step where the biomarkers are then measured, the time of sample collection and storage temperature are used to interpret and correct the obtained results, which may have been affected by for example protein degradation. This makes the actual clinical work less time-depending and time-consuming, and also saves costs. For example, it is not required to do the analysis of each sample on-site where the blood sample is taken, but just to collect samples to be processed and analyzed later on. Also internal logistics in the research unit are thus improved. In addition, blood samples do not require stabilizers for storage purposes, but instead can be stored at for example room temperature as whole blood samples before further analytical use.

Next, the present technology will be described more closely with reference to certain embodiments.

EMBODIMENTS

The present technology relates to the effect of temperature and time during storage and transportation of whole blood samples, when examining a person having or assuming to have the GP biomarkers indicating a *Helicobacter pylori* infection and/or atrophic gastritis with related risks.

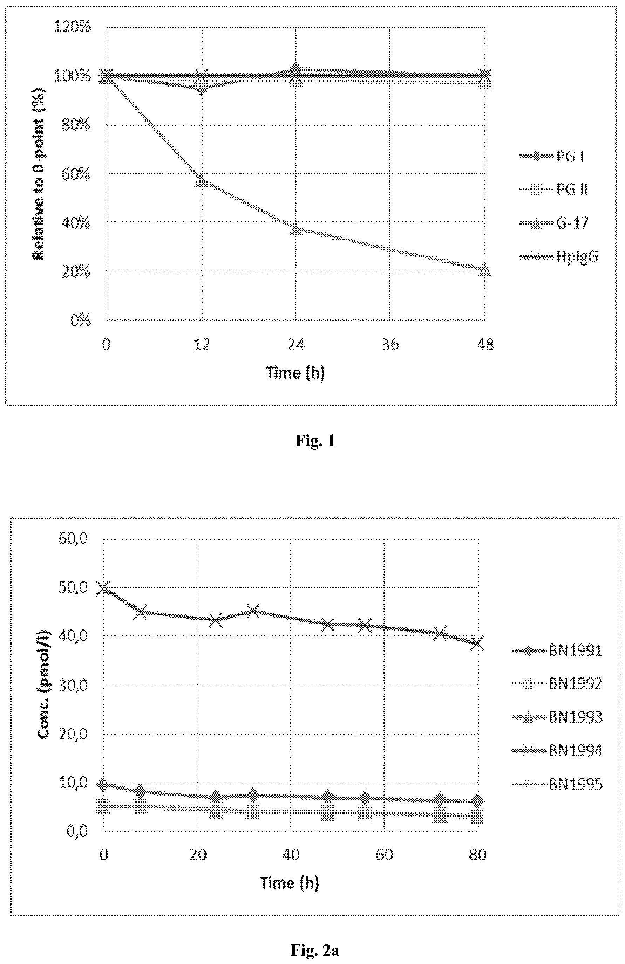

It has been shown that pepsinogen I, pepsinogen II and *Helicobacter pylori* stands well without substantial degradation for few days storage at normal room temperature, unlike gastrin-17, as illustrated in figure 1. In this context different protein degradation events like oxidation, photodegradation, disulfide scrambling, deamidation, aggregation, precipitation, dissociation, fragmentation etc., are not of interest, but simply the effect of storage time and temperature.

Figure 1 is a diagram showing relative concentration change of pepsinogens and gastrin-17, when samples are stored as whole blood at 21 °C with different period of time before analyses.

Figures 2 to 4 illustrate gastrin-17 concentration after storing the samples for 12, 24, 36 and 48 hours at 4 °C, room temperature of 21 °C, and 30 °C. The relative drop of concentration is about 20%, 40% and 80% within 24 hours at 4 °C, 21 °C and 30 °C, respectively.

More precisely, figure 2a is a diagram showing the gastrin-17 concentration of samples stored as whole blood at 4 °C with different period of time before analysis

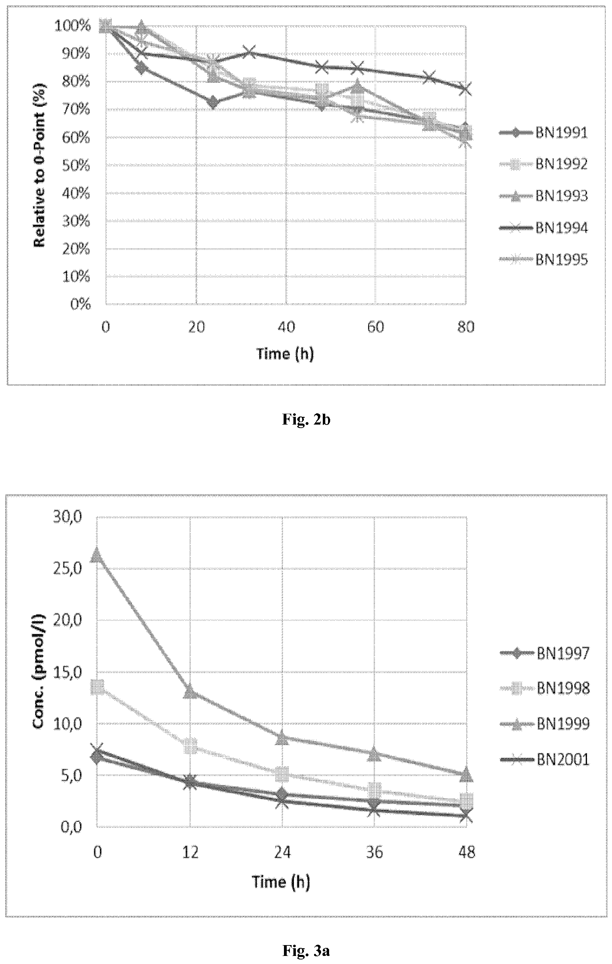

Figure 2b is a diagram showing the gastrin-17 relative concentration change of samples as whole blood at 4 °C with different period of time before analysis.

Figure 3a is a diagram showing the gastrin-17 concentration of samples as whole blood at 21 °C with different period of time before analysis.

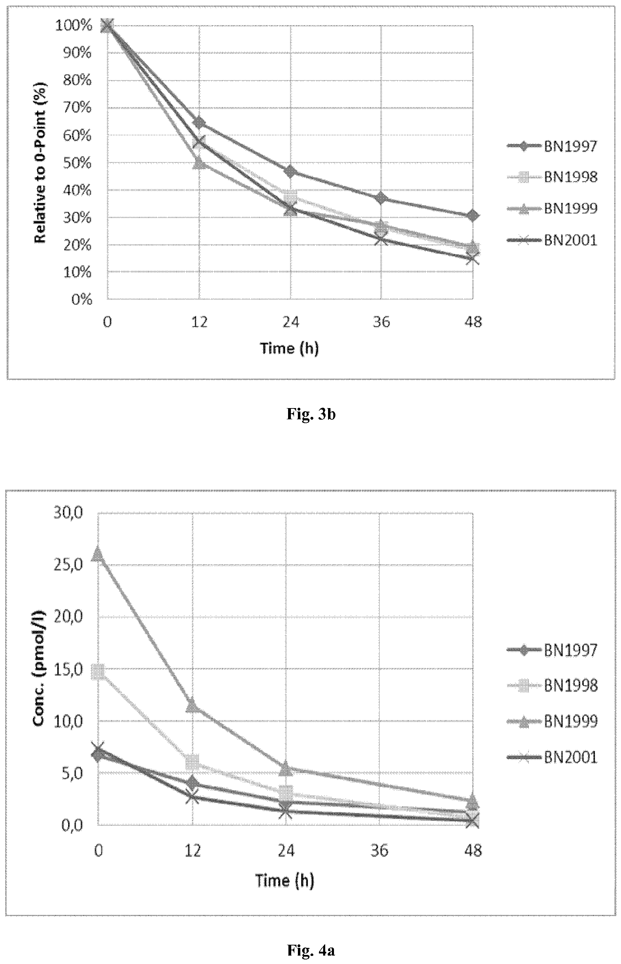

Figure 3b is a diagram showing the gastrin-17 relative concentration change of samples as whole blood at 21 °C with different period of time before analysis.

Figure 4a is a diagram showing the gastrin-17 concentration of samples as whole blood at 30 °C with different period of time before analysis.

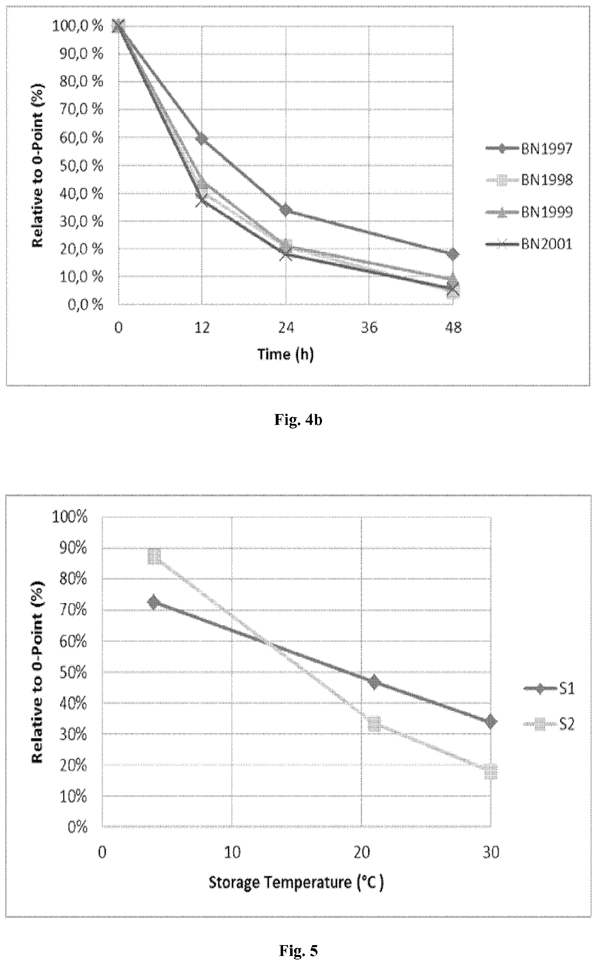

Figure 4b is a diagram showing the gastrin-17 relative concentration change of samples as whole blood at 30 °C with different period of time before analysis.

Storage/transportation temperature has significant effect on the gastrin-17 degradation rate as shown in figure 5. Thus, figure 5 is a diagram showing one day (24 h) storage of whole blood in different temperatures for two different samples.

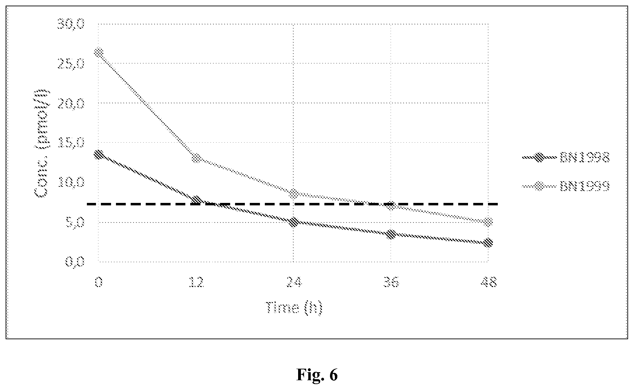

Figure 6 is a diagram showing gastrin-17 concentration of samples stored as whole blood at 30 °C with different period of time before analyses. The dashed line presents gastrin-17 high reference of 7 pmol/l (an example of high reference; any higher concentration indicates low acid).

One embodiment of the present invention is a method for screening of asymptomatic and symptomatic subjects with *Helicobacter pylori* infection, atrophic gastritis with related risks and/or disturbed gastric function, said method including the steps of:

a) recording the collection time of a whole blood sample which has been obtained from a subject,

b) recording the storage temperature of the whole blood sample,

c) quantitatively measuring gastrin-17 concentration of a plasma or serum sample separated from the whole blood sample,

d) determining initial gastrin-17 concentration according to the time between the whole blood sample collection and plasma and/or serum sample analysis at a given storage temperature, and

e) interpreting the structure and/or function of the stomach mucosa based on said comparison.

A method, wherein in addition to gastrin-17 concentration also concentrations of pepsinogen I, pepsinogen II and *Helicobacter pylori* IgG antibodies are determined from the plasma or serum sample, belongs to the scope of the present invention.

One suitable, but not limited to, analytical function for estimating the initial concentration of the samples is as follows. If assumed that the concentration decrease is proportional to the number of molecules

$$-\frac{dN}{dt} \approx N, or -\frac{dN}{N} = kdt,$$

where

N is the number of molecules, k indicates rate at which decrease will take place, and t is the time.

The solution to the 1st order differential equation can be presented as follows:

$$N(t) = N_0 \, e^{-kt},$$

where

$N_0$ is the initial number of molecules i.e. initial concentration of the analyte, and $N(t)$ is the measured number if molecules ($\approx$ concentration).

Because the measured concentration is affected by the storage temperature, the function can be presented by general form:

$$N(t, T) = N_0 \, e^{-kt} f(T),$$

where

T is the storage temperature of the sample.

The concentration half time $t_{1/2}$ can be solved from the formula:

$$k(T) = \frac{t_{\frac{1}{2}}}{\ln(2)} f(T)$$

The half time $t_{\frac{1}{2}}$ and $k(T)$ can be estimated from the measured data.

As the concentration drops monotonically vs time and temperature, and if the concentration is higher than the Limit of Quantification (LoQ), and information of the storage temperature and time of sample collection is known, it is possible to estimate the initial concentration and use that information when interpreting the results.

According to one embodiment of the present invention, high (vs the reference range) gastrin-17 concentration indicates acid-free stomach. If a sample that has been stored for some time before measurement still gives high concentration value, also the initial concentration must have been high. Such a situation is illustrated by the sample BN1999 in the figure 5, assuming that measurement have been carried out next day (24 hours) from sample collection. The interpretation on stored sample results is as valid as if the sample would have been analyzed right after collection.

Further, if it is sure that the temperature chain has not exceeded certain temperature, it can predict concentration vs. reference range, and enable the use of that when interpreting the results. For example, when sample BN1998 in figure 5 has been stored at room temperature (21 °C) before measurement, and the measured value is 5 pmol/1, it can be assumed that the half time is less than 24 hours, and therefore be confident that the initial value has been higher than the example high reference of 7 pmol/l. Thus, also this case indicates lack of acid in the stomach and e.g. with low pepsinogen level will increase the specificity of atrophic gastritis diagnosis.

Furthermore, if it is assumed that the sample has been stored for 24 hours before measurement, and the value is about 2 pmol/l, it indicates that the acid content of the stomach is normal, less than the upper limit of the reference range.

However, if the measured concentration is below the LoQ, it is not possible to predict the initial concentration. In that case the gastrin-17 results cannot be used for the interpretation. The GastroPanel® interpretation must then be made based on the other biomarker values.

According to one embodiment the whole blood sample is collected and stored without stabilizers.

According to one embodiment the time period between whole blood sample collection and further analysis from the plasma or serum sample is between 0 to 120 hours, more preferably between 0 to 72 hours and most suitably between 0 to 48 hours.

According to one embodiment the whole blood sample is collected and stored at temperatures between 0 to 30 °C, for example at 4 °C, 21 °C or 30 °C, before the separation of plasma and/or serum and analysis of the biomarker concentrations.

Preferably, the plasma or serum biomarker concentration is above the limit of quantification, the time between whole blood sample collection and plasma and/or sample analysis is between 0 to 48 hours, and the storage temperature of the whole blood sample is between 0 to 30 °C.

One further embodiment of the present invention is to use the methods as disclosed in a non-invasive test for screening of asymptomatic and symptomatic subjects with *Helicobacter pylori* infection, atrophic gastritis with related risks and/or disturbed gastric function.

It is to be understood that the embodiments of the invention disclosed are not limited to the particular structures, process steps, or materials disclosed herein, but are extended to equivalents thereof as would be recognized by those ordinarily skilled in the relevant arts. It should also be understood that terminology employed herein is used for the purpose of describing particular embodiments only and is not intended to be limiting.

Reference throughout this specification to one embodiment or an embodiment means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment. Where reference is made to a numerical value using a term such as, for example, about or substantially, the exact numerical value is also disclosed.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary. In addition, various embodiments and example of the present invention may be referred to herein along with alternatives for the various components thereof. It is understood that such embodiments, examples, and alternatives are not to be construed as de facto equivalents of one another, but are to be considered as separate and autonomous representations of the present invention.

Furthermore, the described features, structures, or characteristics may be combined in any suitable manner in one or more embodiments. In the following description, numerous specific details are provided to provide a thorough understanding of embodiments of the invention. One skilled in the relevant art will recognize, however, that the invention can be practiced without one or more of the specific details, or with other methods, components, materials, etc. In other instances, well-known structures, materials, or operations are not shown or described in detail to avoid obscuring aspects of the invention.

While the forgoing examples are illustrative of the principles of the present invention in one or more particular applications, it will be apparent to those of ordinary skill in the art that numerous modifications in form, usage and details of implementation can be made without the exercise of inventive faculty, and without departing from the principles and concepts of the invention. Accordingly, it is not intended that the invention be limited, except as by the claims set forth below.

The verbs "to comprise" and "to include" are used in this document as open limitations that neither exclude nor require the existence of also un-recited features. The features recited in depending claims are mutually freely combinable unless otherwise explicitly stated. Furthermore, it is to be understood that the use of "a" or "an", that is, a singular form, throughout this document does not exclude a plurality.

INDUSTRIAL APPLICABILITY

At least some embodiments of the present invention find industrial application for example in screening and examination of patients with dyspepsia, as well as for the screening and detection of atrophic gastritis (AG) with related risks, such as stomach- and oesophageal cancer.

CITATION LIST

Patent literature:

1. WO 2009/053537

Non patent literature:

1. Agréus L, Kuipers EJ, Kupcinskas L, Malfertheiner P, Di Mario F, Leja M, Mahachai V, Yaron N, van Oijen M, Perez Perez G, Rugge M, Ronkainen J, Salaspuro M, Sipponen P, Sugano K and Sung J: Rationale in diagnosis and screening of atrophic gastritis with stomach-specific plasma biomarkers. Scand J Gastroenterol 2012;47:136-147.

2. Storskrubb T, Arc P, Ronkainen J, Sipponen P, Nyhlin H and Talley NJ: Serum biomarkers provide an accurate method for diagnosis of atrophic gastritis in a general population: the Kalixanda study. Scand J Gastroenterol 2008;43:448-1455.

3. Wikström M: Assessment of stomach health by "chemical gastroscopy". Eur Gastroenterol Rev 2012;1-6.

4. Lomba-Viana R, Dinis-Ribeiro M, Fonseca F, Vieira AS, Bento MJ and Lomba-Viana H: Serum pepsinogen test for early detection of gastric cancer in a European country. Eur J Gastroenterol Hepatol 2012;24:37-41.

5. Miki K: Gastric cancer screening using the serum pepsinogen test method. Gastric Cancer 2006;9:245-253.

6. Bornschein J, Selgrad M, Wex T, Kuester D and Malfertheiner P: Serological assessment of gastric mucosal atrophy in gastric cancer. BMC Gastroenterol 201212:10. doi: 10.1186/1471-230X-12-10.

7. Germaná B, Di Mario F, Cavallaro LG, Moussa AM, Lecis P, Liatoupolou S, Comparato G, Carloni C, Bertiato G, Battiestel M, Papa N, Aragona G, Cavestro GM, Ion V, Merli R, Bertolini S, Caruana P and Franzé A: Clinical usefulness of serum pepsinogens I and II, gastrin-17 and anti-Helicobacter pylori antibodies in the management of dyspeptic patients in primary care. Dig Liver Dis 2005;37:501-508.

8. Miki K, Ichinose M, Shimizu A, Huang SC, Oka H, Furihata C, Matsushima T and Takahashi K: Serum pepsinogens as a screening test of extensive chronic gastritis. Gastroenterol Jpn 1987;22:133-141.

9. Samloff IM, Varis K, Ihamaki T, Siurala M and Rotter JI: Relationships among serum pepsinogen I, serum pepsinogen II, and gastric mucosal histology. A study in relatives of patients with pernicious anemia. Gastroenterol 1982;83:204-209.

10. Korstanje A, den Hartog G, Biemond I and Lamers CB: The serological gastric biopsy: a non-endoscopical diagnostic approach in management of the dyspeptic patient: significance for primary care based on a survey of the literature. Scand J Gastroenterol 2002 236 (Suppl): 22-26.

11. Oksanen A, Sipponen P, Miettinen A, Sarna S and Rautelin H: Evaluation of bood tests to normal gastric mucosa. Scand J Gastroenterol 2000;35:791-795.

12. Varis K, Sipponen P, Laxen F, Samloff 1M, Huttunen JK, Taylor PR, and The Helsinki Gastritis Study Group: Implications of serum pepsinogen I in early endoscopic diagnosis of gastric cancer and dysplasia. Scand J Gastroenterol 2000:35:950-956.

13. Dixon MF, Genta RM, Yardley JH and Correa P: Classification and grading of gastritis. The updated Sydney System. International Workshop on the Histopathology of Gastritis, Houston 1994. Am J Surg Pathol 1996;20:1161-1181.

14. Väänänen H, Vauhkonen M, Helske T, et al. Non-endoscopic diagnosis of atrophic gastritis with a blood test. Correlation between gastric histology and serum levels of gastrin-17 and pepsinogen I: a multicenter study. Eur J Gastroenterol Hepatol 2003:15:885-891.

15. Telaranta-Keerie A, Kara R, Paloheimo L, Härkönen M and Sipponen P: Prevalence of undiagnosed advanced atrophic corpus gastritis in Finland: an observational study among 4,256 volunteers without specific complaints. Scand J Gastroenterol 2010;45:1036-1041.

16. Dinis-Ribeiro M, Yamaki G, Miki K, Costa-Pereira A, Matsukawa M and Kurihara M: Meta-analysis on the validity of pepsinogen test for gastric carcinoma, dysplasia or chronic atrophic gastritis screening. J Med Screen 2004;11:141-147.

17. Sipponen P, Ranta P, Helske T, Kääriäinen I, Mäki T and Linnala A. Serum levels of amidated gastrin-17 and pepsinogen I in atrophic gastritis: an observational case-control study. Scand J Gastroenterol 2002;37:785-791.

18. Benberin V, Bektayeva R, Karabayeva R ym. Prevalence of H.pylori infection and atrophic gastritis among ymptomatic and dyspeptic adults in Kazakhstan. A hospital-based screening with a panel of serum biomarkers. Anticancer Res 2013;33:4595-4602.

19. Syrjänen KJ, Sipponen P, Härkönen M, Peetsalu A, Korpela S. Accuracy of GastroPanel testing in detection of atrophic gastritis. Eur J Gastroenterol Hepatol 2015;27:102-104.

20. Misiewicz JJ. The Sydney System: a new classification of gastritis. Introduction. J Gastroenterol Hepatol. 1991; 6:207-208.

21. Sipponen P, Price AB. The Sydney System for classification of gastritis 20 years ago. J Gastroenterol Hepatol. 2011;26: Suppl 1:31-34.

22. Malfertheiner P, Megraud F, O'Morain CA, Atherton J, Axon AT, Bazzoli F, Gensini GF, Gisbert JP, Graham DY, Rokkas T, El-Omar EM, Kuipers EJ. European Helicobacter Study Group. Management of Helicobacter pylori infection - the Maastricht IV/ Florence Consensus Report. Gut 2012;61:646-664.

23. Syrjänen K. A Panel Of Serum Biomarkers (GastroPanel®) in Non-invasive Diagnosis of Atrophic Gastritis. Systematic Review and Meta-analysis. Anticancer Research 36: 5133-5144 (2016).

## Claims

1. A method for screening of asymptomatic and symptomatic subjects with *Helicobacter pylori* infection, atrophic gastritis with related risks and/or disturbed gastric function, the method including the steps of:

    a) recording the collection time of a whole blood sample, which has been obtained from a subject,
    b) recording the storage temperature of the whole blood sample,
    c) quantitatively measuring gastrin-17 concentration of a plasma or serum sample separated from the whole blood sample,
    d) determining initial gastrin-17 concentration according to the time between the whole blood sample collection and plasma and/or serum sample analysis at a given storage temperature, and
    e) interpreting the structure and/or function of the stomach mucosa of the subject based on said determination.

2. The method according to claim 1, wherein in addition to gastrin-17 concentration also concentrations of pepsinogen I, pepsinogen II and *Helicobacter pylori* IgG antibodies are measured from the plasma or serum sample.

3. The method according to claim 1 or 2, wherein the whole blood sample is collected and stored without stabilizers.

4. The method according to any of claims 1 to 3, wherein the whole blood sample is collected and stored at temperatures between 0 to 30 °C before the separation of plasma and/or serum and analysis of the biomarker concentrations.

5. The method according to any of claims 1 to 4 for use in a non-invasive test for screening of asymptomatic and symptomatic subjects with *Helicobacter pylori* infection, atrophic gastritis with related risks and/or disturbed gastric function.

## Patentansprüche

1. Verfahren zum Screening asymptomatischer und symptomatischer Probanden mit *Helicobacter-pylori*-Infektion, atrophischer Gastritis mit damit verbundenen Risiken und/oder gestörter Magenfunktion, wobei das Verfahren die folgenden Schritte umfasst:

    a) Erfassen des Entnahmezeitpunkts einer Vollblutprobe, die einem Probanden entnommen wurde,
    b) Erfassen der Lagertemperatur der Vollblutprobe,
    c) quantitatives Messen der Gastrin-17-Konzentration einer Plasma- oder Serumprobe, die von der Vollblutprobe getrennt wurde,
    d) Bestimmen der anfänglichen Gastrin-17-Konzentration gemäß der Zeit zwischen der Entnahme der Vollblutprobe und der Analyse der Plasma- und/oder Serumprobe bei einer gegebenen Lagertemperatur, und
    e) Interpretieren der Struktur und/oder Funktion der Magenschleimhaut des Probanden basierend auf der Bestimmung.

2. Verfahren nach Anspruch 1, wobei zusätzlich zur Gastrin-17-Konzentration auch Konzentrationen von Pepsinogen I, Pepsinogen II und *Helicobacter-pylori*-IgG-Antikörpern aus der Plasma- oder Serumprobe gemessen werden.

3. Verfahren nach Anspruch 1 oder 2, wobei die Vollblutprobe ohne Stabilisatoren entnommen und gelagert wird.

4. Verfahren nach einem der Ansprüche 1 bis 3, wobei die Vollblutprobe entnommen und bei Temperaturen zwischen 0 bis 30 °C gelagert wird, bevor Plasma und/oder Serum getrennt und die Biomarkerkonzentrationen analysiert werden.

5. Verfahren nach einem der Ansprüche 1 bis 4 zur Verwendung in einem nichtinvasiven Test zum Screening asymptomatischer und symptomatischer Probanden mit *Helicobacter-pylori*-Infektion, atrophischer Gastritis mit damit verbundenen Risiken und/oder gestörter Magenfunktion.

## Revendications

1. Procédé de dépistage de sujets asymptomatiques et symptomatiques atteints d'une infection par *Helicobacter pylori,* d'une gastrite atrophique avec des risques associés et/ou d'une fonction gastrique perturbée, le procédé comprenant les étapes consistant à :

    a) enregistrer l'heure de prélèvement d'un échantillon de sang total, qui a été obtenu d'un sujet,
    b) enregistrer la température de stockage de l'échantillon de sang total,

c) mesurer quantitativement la concentration de gastrine-17 d'un échantillon de plasma ou de sérum séparé de l'échantillon de sang total,

d) déterminer la concentration initiale de gastrine-17 en fonction du temps écoulé entre le prélèvement de l'échantillon de sang total et l'analyse de l'échantillon de plasma et/ou de sérum à une température de stockage donnée, et

e) interpréter la structure et/ou la fonction de la muqueuse gastrique du sujet sur la base de ladite détermination.

2. Procédé selon la revendication 1, dans lequel, en plus de la concentration de gastrine-17, des concentrations d'anticorps de pepsinogène I, pepsinogène II et *Helicobacter pylori* IgG sont également mesurées à partir de l'échantillon de plasma ou de sérum.

3. Procédé selon la revendication 1 ou 2, dans lequel l'échantillon de sang total est collecté et stocké sans stabilisateurs.

4. Procédé selon l'une quelconque des revendications 1 à 3, dans lequel l'échantillon de sang total est collecté et stocké à des températures comprises entre 0 et 30°C avant la séparation du plasma et/ou du sérum et l'analyse des concentrations de biomarqueurs.

5. Procédé selon l'une quelconque des revendications 1 à 4 destiné à être utilisé dans un test non invasif pour le dépistage de sujets asymptomatiques et symptomatiques atteints d'une infection par *Helicobacter pylori,* d'une gastrite atrophique avec des risques associés et/ou d'une fonction gastrique perturbée.

**Fig. 1**

**Fig. 2a**

**Fig. 2b**

**Fig. 3a**

**Fig. 3b**

**Fig. 4a**

**Fig. 4b**

**Fig. 5**

**Fig. 6**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009053537 A

**Non-patent literature cited in the description**

- **AGRÉUS L ; KUIPERS EJ ; KUPCINSKAS L ; MALFERTHEINER P ; DI MARIO F ; LEJA M ; MAHACHAI V ; YARON N ; VAN OIJEN M ; PEREZ PEREZ G.** Rationale in diagnosis and screening of atrophic gastritis with stomach-specific plasma biomarkers. *Scand J Gastroenterol,* 2012, vol. 47, 136-147
- **STORSKRUBB T ; ARC P ; RONKAINEN J ; SIPPONEN P ; NYHLIN H ; TALLEY NJ.** Serum biomarkers provide an accurate method for diagnosis of atrophic gastritis in a general population: the Kalixanda study. *Scand J Gastroenterol,* 2008, vol. 43, 448-1455
- **WIKSTRÖM M.** Assessment of stomach health by "chemical gastroscopy. *Eur Gastroenterol Rev,* 2012, 1-6
- **LOMBA-VIANA R ; DINIS-RIBEIRO M ; FONSECA F ; VIEIRA AS ; BENTO MJ ; LOMBA-VIANA H.** Serum pepsinogen test for early detection of gastric cancer in a European country. *Eur J Gastroenterol Hepatol,* 2012, vol. 24, 37-41
- **MIKI K.** Gastric cancer screening using the serum pepsinogen test method. *Gastric Cancer,* 2006, vol. 9, 245-253
- **BORNSCHEIN J ; SELGRAD M ; WEX T ; KUESTER D ; MALFERTHEINER P.** Serological assessment of gastric mucosal atrophy in gastric cancer. *BMC Gastroenterol,* December 2012, vol. 201212, 10
- **GERMANÁ B ; DI MARIO F ; CAVALLARO LG ; MOUSSA AM ; LECIS P ; LIATOUPOLOU S ; COMPARATO G ; CARLONI C ; BERTIATO G ; BATTIESTEL M.** Clinical usefulness of serum pepsinogens I and II, gastrin-17 and anti-Helicobacter pylori antibodies in the management of dyspeptic patients in primary care. *Dig Liver Dis,* 2005, vol. 37, 501-508
- **MIKI K ; ICHINOSE M ; SHIMIZU A ; HUANG SC ; OKA H ; FURIHATA C ; MATSUSHIMA T ; TAKAHASHI K.** Serum pepsinogens as a screening test of extensive chronic gastritis. *Gastroenterol Jpn,* 1987, vol. 22, 133-141

- **SAMLOFF IM ; VARIS K ; IHAMAKI T ; SIURALA M ; ROTTER JI.** Relationships among serum pepsinogen I, serum pepsinogen II, and gastric mucosal histology. *A study in relatives of patients with pernicious anemia. Gastroenterol,* 1982, vol. 83, 204-209
- **KORSTANJE A ; DEN HARTOG G ; BIEMOND I ; LAMERS CB.** The serological gastric biopsy: a non-endoscopical diagnostic approach in management of the dyspeptic patient: significance for primary care based on a survey of the literature. *Scand J Gastroenterol,* 2002, vol. 236, 22-26
- **OKSANEN A ; SIPPONEN P ; MIETTINEN A ; SARNA S ; RAUTELIN H.** Evaluation of bood tests to normal gastric mucosa. *Scand J Gastroenterol,* 2000, vol. 35, 791-795
- **VARIS K ; SIPPONEN P ; LAXEN F ; SAMLOFF 1M ; HUTTUNEN JK ; TAYLOR PR.** The Helsinki Gastritis Study Group: Implications of serum pepsinogen I in early endoscopic diagnosis of gastric cancer and dysplasia. *Scand J Gastroenterol,* 2000, vol. 35, 950-956
- **DIXON MF ; GENTA RM ; YARDLEY JH ; CORREA P.** Classification and grading of gastritis. The updated Sydney System. International Workshop on the Histopathology of Gastritis, Houston 1994. *Am J Surg Pathol,* 1996, vol. 20, 1161-1181
- **VÄÄNÄNEN H ; VAUHKONEN M ; HELSKE T et al.** Non-endoscopic diagnosis of atrophic gastritis with a blood test. Correlation between gastric histology and serum levels of gastrin-17 and pepsinogen I: a multicenter study. *Eur J Gastroenterol Hepatol,* 2003, vol. 15, 885-891
- **TELARANTA-KEERIE A ; KARA R ; PALOHEIMO L ; HÄRKÖNEN M ; SIPPONEN P.** Prevalence of undiagnosed advanced atrophic corpus gastritis in Finland: an observational study among 4,256 volunteers without specific complaints. *Scand J Gastroenterol,* 2010, vol. 45, 1036-1041

- **DINIS-RIBEIRO M ; YAMAKI G ; MIKI K ; COSTA-PEREIRA A ; MATSUKAWA M ; KURIHARA M.** Meta-analysis on the validity of pepsinogen test for gastric carcinoma, dysplasia or chronic atrophic gastritis screening. *J Med Screen,* 2004, vol. 11, 141-147
- **SIPPONEN P ; RANTA P ; HELSKE T ; KÄÄRIÄINEN I ; MÄKI T ; LINNALA A.** Serum levels of amidated gastrin-17 and pepsinogen I in atrophic gastritis: an observational case-control study. *Scand J Gastroenterol,* 2002, vol. 37, 785-791
- **BENBERIN V ; BEKTAYEVA R ; KARABAYEVA R.** Prevalence of H.pylori infection and atrophic gastritis among ymptomatic and dyspeptic adults in Kazakhstan. A hospital-based screening with a panel of serum biomarkers. *Anticancer Res,* 2013, vol. 33, 4595-4602
- **SYRJÄNEN KJ ; SIPPONEN P ; HÄRKÖNEN M ; PEETSALU A ; KORPELA S.** Accuracy of Gastro-Panel testing in detection of atrophic gastritis. *Eur J Gastroenterol Hepatol,* 2015, vol. 27, 102-104
- **MISIEWICZ JJ.** The Sydney System: a new classification of gastritis. *Introduction. J Gastroenterol Hepatol,* 1991, vol. 6, 207-208
- **SIPPONEN P ; PRICE AB.** The Sydney System for classification of gastritis 20 years ago. *J Gastroenterol Hepatol,* 2011, vol. 26 (1), 31-34
- **MALFERTHEINER P ; MEGRAUD F ; O'MORAIN CA ; ATHERTON J ; AXON AT ; BAZZOLI F ; GENSINI GF ; GISBERT JP ; GRAHAM DY ; ROKKAS T.** *Management of Helicobacter pylori infection - the Maastricht IV/ Florence Consensus Report. Gut,* 2012, vol. 61, 646-664
- **SYRJÄNEN K.** A Panel Of Serum Biomarkers (GastroPanel®) in Non-invasive Diagnosis of Atrophic Gastritis. Systematic Review and Meta-analysis. *Anticancer Research,* 2016, vol. 36, 5133-5144